# DEMANDE DE BREVET EUROPEEN

(11) **EP 4 331 686 A2**
(43) Date de publication de la demande: **06.03.2024**
(21) Numéro de dépôt: 24152640.9
(22) Date de dépôt: 11.03.2020
(51) Int. Cl.: A61Q 19/00

(54) **COMPOSITIONS À BASE D`ALCANES ET D`ESTER STABLES AU STOCKAGE EN TEMPÉRATURE, LEUR UTILISATION COMME AGENTS ÉMOLLIENTS ET ÉMULSIONS LES COMPRENANT**

(30) Priorité: 28.03.2019 FR 1903276
(62) Demande divisionnaire de: 20725876.5
(71) Demandeur: Société d'Exploitation de Produits pour les Industries Chimiques SEPPIC, 75321 Paris cedex 07 (FR); TotalEnergies OneTech, 92400 Courbevoie (FR)
(72) Inventeur: SIGURANI, Séverine, 81100 CASTRES (FR); MIZAEL, Sabrina, 92250 LA GARENNE COLOMBES (FR)
(74) Mandataire: Alatis

(57) **Abrégé**

Composition (C1) comprenant pour 100% de sa masse :
a) de 30% à 80% massique d'au moins un ester choisi parmi les éléments du groupe constitué par :
- les composés de formule (I) :
CH₃- (CH₂)ₓ₁- C(=O)-O-CH₂-CH[O-C(=O)-(CH₂)ₓ₂-CH₃]-CH₂-O-C(=O)-(CH₂)ₓ₃-CH₃ (I) avec x₁, x₂ et x₃ représentant un nombre entier compris entre 6 et 8, et par
- les composés de formule (II) :

R-C(=O)-O-R' (II)

avec :
- R-C(=O) représente un radical acyle linéaire et saturé, comportant de huit à dix atomes de carbone et
- R' représente un radical alkyle linéaire et saturé, comportant de huit à dix-huit atomes de carbone,
et

b) de 20% massique à 70% massique d'un mélange (M1) d'hydrocarbures parmi lesquels au moins 94% massique comportent de quinze à dix-neuf atomes de carbone, ledit mélange (M1) comprenant pour 100% de sa masse:
- Une proportion massique en alcanes ramifiés supérieure ou égale à 90% et inférieure ou égale à 100%;
- Une proportion massique en alcanes linéaires supérieure ou égale à 0% et inférieure ou égale à 10%;
- Une proportion massique en cyclo-alcanes supérieure ou égale à 0% et inférieure ou égale à 1%.
émulsions huile-dans-eau, émulsions eau-dans-huile comprenant une telle composition (C1) et leur utilisation en pharmacie et en cosmétique.

## Description

La présente invention concerne une composition à usage topique comprenant au moins un mélange d'hydrocarbures saturés cycliques ou acycliques, linéaires ou ramifiés et au moins un ester d'acide gras, présentant comme propriétés de rester limpide, incolore, non odorant après stockage à différentes températures.

La présente invention concerne également l'utilisation de ladite composition et les formulations cosmétiques à usage topique la comprenant.

De nombreuses formulations cosmétiques à usage topique ont pour but de protéger la peau, les muqueuses et le cuir chevelu, des agressions et des stresses extérieurs et environnementaux. Par exemple, le consommateur recherche des formulations à appliquer sur la peau qui le protégera des effets néfastes et inesthétiques d'une exposition prolongée aux rayonnements ultra-violets du soleil, ou qui préviendra contre des altérations à l'intégrité de sa peau suite à une exposition de plus en plus fréquentes aux agents polluants présents dans les atmosphères, et plus particulièrement les atmosphères urbaines. Pour répondre à ces demandes visant à maintenir et/ou à restaurer l'intégrité de la peau face à des éléments extérieurs identifiés ou au vieillissement naturel, les industries de la cosmétique et de la dermocosmétique ont développé ces dernières décennies de nouveaux ingrédients spécifiques pour améliorer les performances requises par les consommateurs.

D'autre part, le consommateur est aussi en attente de propriétés d'ordres sensorielles et esthétiques apportées par les compositions cosmétiques et dermocosmétiques, qui lui procurent à la fois des sensations de bien-être pendant et après l'application sur la peau, et également une identification à un aspect extérieur de la formulation cosmétique ou dermocosmétique, renvoyant à un critère de qualité. Ainsi, les consommateurs sont en recherche de compositions cosmétiques ou dermocosmétiques qui procurent un confort cutané pendant et après l'application sur la peau. Cette sensation de confort est renforcée lorsque l'étape d'étalement de la composition cosmétique ou dermo-cosmétique est aisée, à savoir lorsque sa durée est réduite ou plus précisément n'est pas prolongée suite à des résistances sur la peau lors dudit étalement et/ou lorsque le consommateur doit appliquer une force de cisaillement sur la peau élevée, se traduisant également par une vitesse plus élevée d'application à l'étalement et/ou par l'apposition d'une force à intensité plus élevée lors de la dite phase d'étalement.

Dans des cas spécifiques, comme par exemple ceux des formulations cosmétiques ou dermocosmétiques destinées à la prévention contre les effets non désirés (rougeurs, érythèmes et brûlures) des rayonnements ultra-violets du soleil sur la peau ou ceux des crèmes destinées aux traitements anti-inflammatoires et antirhumatismales par voie locale, qui se caractérisent par des difficultés de mise en oeuvre lors de l'étalement, il a été observé que les consommateurs sont moins rigoureux dans la mise en oeuvre des procédures d'application desdits produits de protection ou de soin. Ils appliquent ainsi souvent trop peu de produit et/ou en une fréquence pas assez soutenue et il en résulte alors une moindre protection de la peau que celle prévue dans la notice et/ou sur le packaging. Ainsi, pour encourager une meilleure et plus fréquente application de ces formulations de prévention et de protection de la peau, il est important que lesdites formulations présentent des propriétés sensorielles agréables et puissent s'étaler sur la peau de façon homogène, rapide et sans impliquer une intensité d'application trop importante. D'autre part, les consommateurs sont à la recherche de formulations cosmétiques ou dermocosmétiques qui n'altèrent pas l'aspect extérieur de la peau, comme par exemple en laissant un résiduel huileux sous la forme d'un léger film et, a contrario, une formulation cosmétique ou dermocosmétique conférant un aspect mat à la peau sera préférée et recherchée.

Prenant en considération ces exigences, la personne du métier dispose d'une catégorie d'ingrédients nommés « émollients », constituée par des substances chimiques et des compositions chimiques, qui peuvent être associées aux autres ingrédients présents dans des formulations cosmétiques ou dermocosmétiques à usage topique.

Dans le cadre de la présente invention, par « agent émollient », on désigne des substances chimiques ou des compositions chimiques qui confèrent à la formulation les contenant, les propriétés d'assouplir, d'adoucir, d'amollir la surface de la peau humaine après application. De tel « agents émollients » sont décrits dans l'ouvrage "International Cosmetic Ingrédient Dictionary and Handbook Vol. 4 (9th ed. 2002)", et plus particulièrement aux pages 2930-2936. La divulgation de "International Cosmetic Ingrédient Dictionary and Handbook Vol. 4, pages 2930-2936" est incorporée par référence dans la présente demande.

La demande de brevet européen publiée sous le numéro EP 2 644 188 A1 divulgue des émulsions de type huile-dans-eau présentant des propriétés d'étalement sur la peau améliorées comprenant une combinaison d'au moins une résine de silicone réticulée et non émulsionnante, au moins un alcool polyvinylique, un épaississant de type polyacrylamide, une huile choisie parmi les éléments du groupe constitué par les huiles végétales de type triglycéridiques, les cires, les alcools gras éthoxylés, les esters d'acides gras, les acides gras, les alcools gras, les huiles de silicone et les huiles perfluorées.

La demande internationale publiée sous le numéro WO 2011/065771 divulgue et enseigne l'étalement et la douceur procurée lors de l'application d'une émulsion de type eau-dans-huile-dans-eau, préparée à partir d'une émulsion de type eau-dans-huile comprenant un émulsionnant sur base silicone, émulsionnant de type di-polyhydroxyalkylate, un épaississant minéral de type hectorite, et une huile polaire.

Ces solutions de l'état de la technique décrivent une amélioration des propriétés d'étalement d'une émulsion en mettant en oeuvre des composés siliconés et d'autres tensioactifs pour lesquels l'homme du métier recherche une alternative dans une démarche de prise en compte d'un développement durable, et plus particulièrement dans une démarche d'utiliser des ingrédients n'émettant pas et/ou dont la fabrication n'implique pas de composés volatiles organiques (VOC), et/ou d'utiliser des ingrédients biodégradables selon les normes réglementaires en vigueur et/ou d'utiliser des ingrédients d'origine végétale et non plus fossile.

De façon plus précise, les dérivés de silicone, comme les substances et compositions chimiques de la famille des polysiloxanes sont connues pour procurer des propriétés sensorielles améliorées aux émulsions huile-dans-eau et notamment en termes de facilité d'étalement et de limitation de résidus lipides sur la peau après application. Cependant, les caractéristiques environnementales associées à ces ingrédients ont nécessité la recherche de substituts qui procurent des propriétés sensorielles similaires tout en ayant des caractéristiques environnementales conformes aux réglementations en vigueur et à venir, et conformes aux exigences des consommateurs en la matière.

Une alternative partiellement satisfaisante a été mise en évidence, par l'utilisation de compositions d'alcanes comprenant des quantités importantes de cyclo-alcanes pour préparer des émulsions cosmétiques huile-dans-eau ; lesdits mélanges d'alcanes présentant des propriétés satisfaisantes de biodégradabilité et des propriétés sensorielles satisfaisantes mais jugées perfectibles.

De récentes avancées ont été divulguées dans la demande internationale de brevet publiée sous le numéro WO2018/109354 A1, et consistent en l'utilisation d'un mélange d'hydrocarbures saturés cycliques ou acycliques, linéaires ou ramifiés comportant de quinze à dix-neuf atomes de carbone, pour préparer des émulsions de type huile-dans-eau dont les propriétés sensorielles et esthétiques, et plus particulièrement les propriétés d'étalement, de consistance et de richesse sont améliorées.

Cependant, la commercialisation mondiale de ces mélanges d'hydrocarbures saturés cycliques ou acycliques, linéaires ou ramifiés comportant de quinze à dix-neuf atomes de carbone, se heurte à des contraintes douanières (Directives 2008/118/CE et 2003/96/CE). En effet, ces mélanges sont assujettis à la réglementation des accises en Europe en étant assimilés à des carburants ou des combustibles de chauffage. Pour éviter que cette réglementation s'applique à un mélange d'agents émollients, il s'avère nécessaire que ledit mélange comprenne une teneur massique inférieure ou égale à 70% d'hydrocarbures saturés cycliques ou acycliques, linéaires ou ramifiés comportant de quinze à dix-neuf atomes de carbone.

Par conséquent, il existe un besoin de mettre au point des agents émollients :
- Qui conservent leurs caractéristiques organoleptiques lors de leur stockage à différentes températures (25°C et 45°C) pendant une durée définie (trois mois), et plus particulièrement qui restent monophasiques, limpides, incolores et inodores après de telles conditions de stockage, et
- Qui permettent, par leur incorporation dans des formulations cosmétiques ou dermocosmétiques, d'en améliorer les propriétés sensorielles, et
- Dont la constitution n'implique pas une classification douanière nécessitant le paiement d'accise Directives 2008/118/CE et 2003/96/CE.

Les inventeurs ont donc cherché à développer une nouvelle solution pour améliorer les propriétés sensorielles d'émulsions de type huile-dans-eau et de type eau-dans-huile à usage topique, n'employant pas nécessairement de dérivés siliconés, mais mettant en oeuvre des compositions chimiques d'origines végétales et/ou biodégradables.

C'est pourquoi selon un premier aspect, l'invention a pour objet une composition (C1) comprenant pour 100% de sa masse :
a) de 30% massique à 80% massique, de préférence de 31% massique à 80% massique, et plus préférentiellement de 31% massique à 50% massique d'au moins un ester choisi parmi les éléments du groupe constitué par :
   - les composés de formule (I) :

      CH₃- (CH₂)₁- C(=O)-O-CH₂-CH[O-C(=O)-(CH₂)ₓ₂-CH₃]-CH₂-O-C(=O)-(CH₂)ₓ₃-CH₃ (I)

      avec x₁, x₂ et x₃ représentant un nombre entier compris entre 6 et 8, et par
      - les composés de formule (II) :

         R-C(=O)-O-R' (II)

         avec :
         R-C(=O) représente un radical acyle comportant de huit à dix atomes de carbone, et
         R' représente un radical alkyle comportant de un à vingt-deux atomes de carbone, et
b) de 20% massique à 70% massique, de préférence de 20% massique à 69% massique, et encore plus particulièrement de 50% massique à 69% massique d'au moins un mélange (M1) d'hydrocarbures parmi lesquels au moins 94% massique comportent de quinze à dix-neuf atomes de carbone.

Notons que :
- x₁, x₂ et x₃ peuvent être identiques ou différents,
- les radicaux acyle et alkyle peuvent être linéaire ou ramifié, saturé ou insaturé, et
- les hydrocarbures du mélange (M1) peuvent être cycliques ou acycliques, linéaires ou ramifiés.

Selon le cas, la composition (C1) selon l'invention peut présenter une ou plusieurs des caractéristiques suivantes :
- dans la formule (I), x₁, x₂ et x₃ représentent des nombres entiers identiques égaux à 6, 7 ou 8.
- dans la formule (I), x₁, x₂ et x₃ représentent un nombre entier identique et égal à 6. Selon cet aspect particulier, la formule (I) représente le glycéryl trioctanoate (numéro CAS = 538-23-8). - dans la formule (I), x₁, x₂ et x₃ représentent un nombre entier identique et égal à 8. Selon cet aspect particulier, la formule (I) représente le glycéryl tridécanoate (numéro CAS = 621-71-6).
- la composition (C1) comprend un mélange de deux composés de formule (I), de préférence un mélange de glycéryl trioctanoate et de glycéryl tridécanoate, et plus préférentiellement un mélange équi-massique de glycéryl trioctanoate et de glycéryl tridécanoate.
- dans la formule (II), R-C(=O) représente un radical acyle linéaire et saturé comportant de huit à dix atomes de carbone.
- dans la formule (II), R' représente un radical alkyle linéaire et saturé, comportant de un à vingt-deux atomes de carbone, de préférence de trois à vingt-deux atomes de carbone, plus préférentiellement de trois à dix-huit atomes de carbone, et encore plus préférentiellement de huit à dix-huit atomes de carbone.
- les composés de formule (II) sont choisis parmi l'octanoate d'éthyle, l'octanoate de propyle, l'octanoate d'isopropyle, l'octanoate de butyle, l'octanoate de pentyle, l'octanoate d'hexyle, l'octanoate d'octyle, l'octanoate de nonyle, l'octanoate de décyle, l'octanoate d'undécyle, l'octanoate de dodécyle, l'octanoate de tétradécyle, l'octanoate d'hexadécyle, l'octanoate d'octadécyle, le nonanoate d'éthyle, le nonanoate de propyle, le nonanoate d'isopropyle, le nonanoate de butyle, le nonanoate de pentyle, le nonanoate d'hexyle, le nonanoate d'octyle, le nonanoate de nonyle, le nonanoate de décyle, le nonanoate d'undécyle, le nonanoate de dodécyle, le nonanoate de tétradécyle, le nonanoate d'hexadécyle, le nonanoate d'octadécyle, le décanoate d'éthyle, le décanoate de propyle, le décanoate d'isopropyle, le décanoate de butyle, le décanoate de pentyle, le décanoate d'hexyle, le décanoate d'octyle, le décanoate de nonyle, le décanoate de décyle, le décanoate d'undécyle, le décanoate de dodécyle, le décanoate de tétradécyle, le décanoate d'hexadécyle, le décanoate d'octadécyle.
- dans la formule (II), R-C(=O) représente un radical acyle linéaire et saturé, comportant de huit à dix atomes de carbone et R' représente un radical alkyle linéaire et saturé, comportant de huit à dix-huit atomes de carbone.
- dans la formule (II), R-C(=O) représente un radical acyle linéaire et saturé choisi parmi les éléments du groupe constitué par le radical octanoyle, le radical nonanoyle et le radical décanoyle, et R' représente un radical alkyle linéaire et saturé choisi parmi les éléments du groupe constitué par le radical n-octyle, le radical n-décyle, le radical n-dodécyle, le radical n-tétradécyle, le radical n-hexadécyle et le radical n-octadécyle.
- la formule (II) représente un des éléments choisi parmi les membres du groupe constitué par l'octanoate d'octyle, l'octanoate de nonyle, l'octanoate de décyle, l'octanoate d'undécyle, l'octanoate de dodécyle, l'octanoate de tétradécyle, l'octanoate d'hexadécyle, l'octanoate d'octadécyle, , le décanoate d'octyle, le décanoate de nonyle, le décanoate de décyle, le décanoate d'undécyle, le décanoate de dodécyle, le décanoate de tétradécyle, le décanoate d'hexadécyle, le décanoate d' octadécyle.
- la formule (II) représente le produit de la réaction d'estérification entre un mélange équimolaire d'acide octanoïque et d'acide décanoïque et d'alcool de coco.
- la formule (II) représente le produit commercialisé sous le nom INCI « coco caprylate caprate » (numéro CAS = 95912-86-0).
- ledit mélange (M1) comprend pour 100% de sa masse une proportion massique en alcanes ramifiés supérieure ou égale à 80 % et inférieure ou égale à 100%, de préférence supérieure ou égale à 90%; une proportion massique en alcanes linéaires supérieure ou égale à 0% et inférieure ou égale à 15%, de préférence inférieure ou égale à 10%; et une proportion massique en cyclo-alcanes supérieure ou égale à 0% et inférieure ou égale à 5%, de préférence inférieure ou égale à 1%.
- ledit mélange (M1) est un mélange d'hydrocarbures saturés commercialisé sous le nom de marque Emogreen^{™}L15 comprenant pour 100% de sa masse: 3,7% d'alcanes linéaires comportant de quinze à dix-neuf atomes de carbone, 96% d'iso-alcanes comportant de quinze à dix-neuf atomes de carbone, et 0,3% de cyclo-alcanes comportant de quinze à dix-neuf atomes de carbone.
- ledit mélange (M1) est un mélange d'hydrocarbures saturés commercialisé sous le nom de marque Emogreen^{™}L19 comprenant pour 100% de sa masse: 3,8% d'alcanes linéaires comportant de quinze à dix-neuf atomes de carbone, 96,2 % d'iso-alcanes comportant de quinze à dix-neuf atomes de carbone, et une proportion massique inférieure à 0,1% de cyclo-alcanes comportant de quinze à dix-neuf atomes de carbone ;
- ledit mélange (M1) est un mélange d'hydrocarbures saturés commercialisé sous le nom de marque Emogreen^{™}L19 comprenant également pour 100% de sa masse, 15% à 20% massique d'alcanes (linéaires, iso-alcanes et cyclo-alcanes) comportant 17 atomes de carbone, 70% à 75% massique d'alcanes (linéaires, iso-alcanes et cyclo-alcanes) comportant 18 atomes de carbone, 4% à 6% massique d'alcanes (linéaires, iso-alcanes et cyclo-alcanes) comportant 19 atomes de carbone.

Par « alcool de coco », on entend au sens de la présente invention le produit de l'hydrogénation d'un mélange d'acides gras provenant de la saponification d'une huile de noix de coco et qui comprend pour 100% de sa masse :
- De 5% à 10% massique d'acide octanoïque
- De 5% à 10% massique d'acide décanoïque
- De 43% à 53% massique d'acide dodécanoïque
- De 15% à 21% massique d'acide tétradécanoïque
- De 7% à 21 % massique d'acide hexadécanoïque
- De 2% à 4% massique d'acide octadécanoïque.

Par « alcanes linéaires » présents dans le mélange (M₁) compris dans la composition (C1) objet de la présente invention et comportant de quinze à dix-neuf atomes de carbone, on désigne plus particulièrement au sens de la présente invention les éléments choisis parmi le groupe constitué par le n-pentadécane, le n-hexadécane, le n-heptadécane, le n-octadécane et le n-nonadécane.

Par « alcanes ramifiés » (ou iso-alcanes) présents dans le mélange (M₁) compris dans la composition (C1) objet de la présente invention et comportant de quinze à dix-neuf atomes de carbone, on désigne plus particulièrement au sens de la présente invention les éléments choisis parmi le groupe constitué par le 2-méthyl tétradécane (ou isopentadécane), le 2-méthyl pentadécane (ou isohexadécane), le 2-méthyl hexadécane (ou isoheptadécane), le 2-méthyl heptadécane (ou isooctadécane) et le 2-méthyl octadécane (ou isononadécane).

Par cyclo-alcanes présents dans le mélange (M₁) compris dans la composition (C1) objet de la présente invention et comportant de quinze à dix-neuf atomes de carbone, on désigne plus particulièrement au sens de la présente invention des hydrocarbures saturés comprenant au moins un groupe hydrocarboné cyclique saturé optionnellement substitué par un ou plusieurs radicaux alkyles linéaires ou ramifies.

Notons que la composition (C1) objet de la présente invention et telle que définie ci-dessus, peut-être préparée par mélange d'au moins un ester choisi parmi le groupe constitué par les composés de formule (I) et les composés de formule (II) avec un mélange (M1) tel que défini précédemment, à une température comprise entre 20°C et 60°C, et sous agitation mécanique muni d'un mobile de type ancre ou turbine, à une vitesse comprise entre 20 tours/minute et 500 tours/minute.

L'invention a aussi pour objet une composition (E1) à usage topique se présentant sous la forme d'une émulsion de type huile-dans-eau, caractérisée en ce qu'elle comprend pour 100% de sa masse :
a) De 50% à 90% massique, de préférence de 60% à 90% massique et encore plus préférentiellement de 70% à 90% massique d'une phase aqueuse (A1) cosmétiquement acceptable,
b) De 10% à 50% massique, de préférence de 10% à 40% massique et encore plus préférentiellement de 10% à 30% massique d'une phase grasse (G1) comprenant pour 100% de sa masse :
   bu) De 10% à 80% massique, de préférence de 10% à 70% massique d'une composition (C₁) selon l'invention;
   b₁₂) De 0,5% à 20% massique, de préférence de 1% à 15% d'au moins un agent tensioactif de type huile-dans-eau (S1),
   b₁₃) De 0% à 89,5% massique, d'au moins une huile et/ou une cire de composition différente de la composition (C₁),
   avec la somme des proportions massiques de b₁₁), b₁₂) et b₁₃) égale à 100% de la phase grasse (G1).

L'expression "à usage topique" utilisée dans la définition de la composition (E1) se présentant sous la forme d'une émulsion de type huile-dans-eau telle que définie ci-dessus, signifie que ladite composition est mise en oeuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

L'expression "cosmétiquement acceptable" utilisée dans la définition de la phase aqueuse (A₁) de la composition (E1) se présentant sous la forme d'une émulsion de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions objet de l'invention peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

Par « huile » présente dans la phase grasse (G₁) de la composition (E1) se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse (G₁) de la composition (E1) se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect solide à une température de 45°C.

Par « agent tensioactif de type huile-dans-eau (S1)» présent dans la phase grasse (G₁) de la composition (E1) se présentant sous la forme d'une émulsion de type huile-dans-eau telle que définie précédemment, on désigne au sens de la présente invention, la substance chimique ou le mélange de substances chimiques qui permet de stabiliser les gouttelettes de ladite phase grasse (G₁) en dispersion dans la phase aqueuse continue (A₁).

Comme agent tensioactif de type huile-dans-eau (S1) présent dans la phase grasse (G1) de l'émulsion (E₁) de type huile-dans-eau telle que définie précédemment, on peut citer par exemple:
- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol;

Par "phase grasse (G1)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires telles que définies précédemment et ne répondant pas à la définition de la composition (C1). Parmi les éléments constitutifs de la phase grasse (G1), on peut citer:
- Les huiles d'origine animale, telles que le squalène ou le squalane;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de mais, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes;
- Les huiles végétales éthoxylées;
- Les huiles synthétiques comme les esters d'acides gras ne répondant pas à la définition de la formule (II) tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, les esters dérivés d'acide lanolique, tels que le lanolate d'isopropyle, le lanolate d'isocétyle, le triheptanoate de glycérol, les alkylbenzoates, les huiles hydrogénées, les polyalphaoléfines, les polyoléfines comme le polyisobutène, le polydécène hydrogéné ou le polyisobutène hydrogéné, commercialisé en France par la société Ets B. Rossow et Cie sous le nom PARLEAM - POLYSYNLANE™, cité dans: Michel and Irene Ash; Thesaurus of Chemical Products, Chemical Publishing Co, Inc. 1986 Volume I, page 211 (ISBN 0 7131 3603 0);
- Les huiles de silicone comme les diméthylpolysiloxanes, les méthylphényl-polysiloxanes, les silicones modifiées par des amines, les silicones modifiées par des acides gras, les silicones modifiées par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.
- Des cires comme la cire d'abeille, la cire de carnauba, la cire de candelilla, la cire d'ouricoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de lignite, les cires microcristallines, la cire de lanoline; I'ozokérite; la cire de polyéthylène, les cires de silicone; les cires végétales; les alcools gras et les acides gras solides à température ambiante; les glycérides solides à température ambiante.

Selon un aspect particulier, dans la composition (E1) objet de la présente invention, la phase grasse (G1) comprend en outre pour 100% de sa propre masse :
b₁₄) de 5% à 30% massique, plus particulièrement de 5% à 25% massique, et encore plus particulièrement de 10% à 25% massique d'au moins un agent de protection contre les rayonnements ultra-violets du soleil,
avec la somme des proportions massiques de b₁₁), b₁₂), b₁₃) et b₁₄) égale à 100% de la phase grasse (G1).

L'agent de protection contre les rayonnements ultra-violets du soleil sera de préférence choisi parmi les éléments du groupe constitué par dioxyde de titane, la 2,4-dihydroxy benzophénone, le 2-(4-diethylamino-2-hydroxybenzoyl) benzoic acid hexyl ester, le 2,4-bis{[4-(2-ethylhexyloxy)-2-hydroxy] phenyl}-6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

Par agent de protection contre les rayonnements ultra-violets du soleil, on désigne notamment dans la définition de la composition (E1) à usage topique se présentant sous la forme d'une émulsion de type huile-dans-eau et objet de la présente invention, les pigments, les filtres organiques solaires et les filtres inorganiques solaires.

Comme pigments utilisés comme agent de protection contre les rayonnements ultra-violets du soleil, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil, il y a par exemple:
- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbomylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil, il y a par exemple: les oxydes de titane, les oxydes de zinc, l'oxyde de cérium, l'oxyde de zirconium, les oxydes de fer j aune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Selon un autre aspect, l'invention a pour objet une composition (E1) selon l'invention pour protéger la peau humaine contre les effets inesthétiques de l'exposition aux rayonnements ultra-violets du soleil, et plus particulièrement contre les rougeurs.

Selon un autre aspect, l'invention a pour objet une composition (E1) selon l'invention pour traiter ou ralentir une altération de la peau due à des brûlures, des coups de soleil, des érythèmes ou des cancers.

Selon un aspect plus particulier, l'invention a pour objet une composition (E1) telle que définie précédemment caractérisée en ce que la phase grasse (G1) comprend en outre au moins pour 100% de sa propre masse :
b₁₄) De 5% à 15% massique d'au moins un pigment coloré (PC),
avec la somme des proportions massiques de b₁₁), b₁₂), b₁₃) et b₁₄) égale à 100% de la phase grasse (G1).

L'invention a pour autre objet une composition (E2) à usage topique se présentant sous la forme d'une émulsion de type eau-dans-huile, caractérisée en ce qu'elle comprend pour 100% de sa masse :
a) De 60% à 98% massique, de préférence de 60% à 95% massique, préférentiellement de 60% à 90% massique, et encore plus préférentiellement de 60% à 85% massique d'une phase aqueuse (A2) cosmétiquement acceptable,
b) De 2% à 40% massique, de préférence de 5% à 40% massique, plus préférentiellement de 10% à 40% massique, et encore plus préférentiellement de 15% à 40% massique d'une phase grasse (G2) comprenant pour 100% de sa masse :
   b₂₁) De 10% à 80% massique, de préférence de 10% à 70% massique d'une composition (C₁) telle que définie à l'une ou quelconque des revendications 1 à 3;
   b₂₂) De 0,5% à 20% massique, de préférence de 1% à 15% massique d'au moins un agent tensioactif de type eau-dans-huile (S2),
   b₂₃) De 0% à 89,5% massique, d'au moins une huile et/ou une cire de composition différente de la composition (C₁), et
   avec la somme des proportions massiques de b₂₁), b₂₂) et b₂₃) égale à 100% de la phase grasse (G2).

Par « huile » présente dans la phase grasse (G₂) de la composition (E2) se présentant sous la forme d'une émulsion de type eau-dans-huile telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse (G₂) de la composition (E2) se présentant sous la forme d'une émulsion de type eau-dans-huile telle que définie précédemment, on désigne au sens de la présente invention les substances chimiques ou les mélanges de substances chimiques insolubles dans l'eau, et se présentant sous un aspect solide à une température de 45°C.

Parmi les tensioactifs émulsionnants de type eau-dans-huile (S2) susceptibles d'être utilisés dans le cadre de la présente invention, on citera notamment les lipoaminoacides et leurs sels ; les lipopeptides et leurs sels; les esters de sorbitan comme par exemple le laurate de sorbitan, le palmitate de sorbitan, le stéarate de sorbitan, l'oléate de sorbitan, le sesquioléate de sorbitan, le trioléate de sorbitan l'isostéarate de sorbitan ; les esters de polyglycérol comme par exemple le laurate de glycérol, le palmitate de glycérol, le stéarate de glycérol, l'oléate de glycérol, le sesquioléate de glycérol, le trioléate de glycérol l'isostéarate de glycérol, le laurate de diglycérol, le palmitate de diglycérol, le stéarate de diglycérol, l'oléate de diglycérol, le sesquioléate de diglycérol, le trioléate de diglycérol l'isostéarate de diglycérol ; les alkylpolyglycosides, les compositions d'alkylpolyglycosides et d'alcools gras, les esters de polyglycérols ou de polyglycols ou de polyols tels que les polyhydroxystéarates de polyglycols ou de polyglycérol comme par exemple les produits dénommés HYPERMER^{®} B246, ARLACEL^{®} P135 commercialisés par la société UNIQEMA, le produit dénommé DEHYMULS^{®} PGPH commercialisé par la société COGNIS, le produit dénommé DECAGLYN^{®} 5HS commercialisé par la société NIKKO ; les copolymères polyéthylèneglycol-alkylglycol comme le PEG-45 dodécylglycol copolymère tel que le produit commercialisé sous la dénomination ELFACOS ST 9^{®} par la société AKZO ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate ; le polyoxystéarate d'aluminium, tel que par exemple le produit commercialisé sous la dénomination MANALOX^{®} par la société RHODIA ; le stéarate de magnésium ; le stéarate d'aluminium.

Selon un aspect plus particulier, l'agent émulsionnant de type eau-dans-huile (S2) est choisi parmi les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés, les copolymères polyéthylèneglycol-alkylglycols.

Par « phase aqueuse (A2) cosmétiquement acceptable », on désigne au sens de la présente invention une phase aqueuse ayant la même définition que la phase aqueuse (A1).

Selon un aspect particulier, dans la composition (E1) objet de la présente invention, la phase grasse (G2) comprend en outre pour 100% de sa propre masse :
b₂₄) de 5% à 30% massique, plus particulièrement de 5% à 25% massique, et encore plus particulièrement de 10% à 25% massique d'au moins un agent de protection contre les rayonnements ultra-violets du soleil,
avec la somme des proportions massiques de b₂₁), b₂₂), b₂₃) et b₂₄) égale à 100% de la phase grasse (G2).

L'agent de protection contre les rayonnements ultra-violets du soleil sera de préférence choisi parmi les éléments du groupe constitué par dioxyde de titane, la 2,4-dihydroxy benzophénone, le 2-(4-diethylamino-2-hydroxybenzoyl) benzoic acid hexyl ester, le 2,4-bis{[4-(2-ethylhexyloxy)-2-hydroxy] phenyl}-6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

Selon un autre aspect, l'invention a pour objet une composition (E2) selon l'invention pour protéger la peau humaine contre les effets inesthétiques de l'exposition aux rayonnements ultra-violets du soleil, et plus particulièrement contre les rougeurs.

Selon un autre aspect, l'invention a pour objet une composition (E2) selon l'invention pour traiter ou ralentir une altération de la peau due à des brûlures, des coups de soleil, des érythèmes ou des cancers.

Selon un aspect plus particulier, l'invention a pour objet une composition (E2) telle que définie précédemment caractérisée en ce que la phase grasse (G2) comprend en outre au moins pour 100% de sa propre masse :
b₂₄) De 5% à 15% massique d'au moins un pigment coloré (PC),
avec la somme des proportions massiques de b₂₁), b₂₂), b₂₃) et b₂₄) égale à 100% de la phase grasse (G2).

De préférence, le pigment coloré (PC) est choisi parmi les éléments du groupe constitué par, les pigments minéraux, les pigments organiques et les pigments nacrés.

Dans le cas où le pigment coloré (PC) est un pigment minéral, il sera de préférence choisi parmi les éléments du groupe constitué par le dioxyde de titane (rutile ou anatase), éventuellement traite en surface; les oxydes de fer noir, jaune, rouge et brun ; le violet de manganèse ; le bleu outremer ; l'oxyde de chrome éventuellement hydraté; le bleu ferrique.

Dans le cas où le pigment coloré (PC) est un pigment organique, il sera de préférence sélectionné parmi les éléments du groupe constitué par le pigment D&C red, le pigment D&C orange, le pigment D&C yellow, le noir de carbone, les laques à base de carmin de cochenille.

Dans le cas où le pigment coloré (PC) est un pigment nacré, il sera de préférence sélectionné parmi les éléments du groupe constitué par les pigments nacrés blancs, comme par exemple que le mica-titane avec des oxydes de fer, le mica-titane avec du bleu ferrique ou de l'oxyde de chrome, le mica-titane avec un pigment organique, les pigments à base d'oxychlorure de bismuth.

L'invention a aussi pour objet un procédé de maquillage caractérisé en ce qu'il comprend une étape d'application sur ladite peau humaine d'une composition (E2) telle que définie précédemment. Ladite étape d'application de la composition à usage topique (F) sur la peau peut être réalisée à l'aide des doigts, ou d'un applicateur comme par exemple un pinceau ou une éponge.

Selon un autre aspect, l'invention a aussi pour objet une composition (E2) à usage topique se présentant sous la forme d'une émulsion de type eau-dans-huile et telle que définie précédemment, caractérisée en ce que la phase aqueuse (A2) comprend pour 100% de sa propre masse de 0,5% massique à 10% massique, de préférence de 0,5% massique à 5% massique, et encore plus préférentiellement de 0,5% massique à 3% massique d'au moins un polymère polyélectrolyte anionique réticulé (P).

Par polyélectrolyte anionique réticulé (P), on désigne, dans la définition de la composition (E2) à usage topique se présentant sous la forme d'une émulsion de type eau-dans-huile et objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Selon un aspect particulier de la présente invention, le polyélectrolyte anionique réticulé (P) présent dans la composition (E2), comprend une proportion supérieure ou égale à 25% molaire d'unités monomériques issues de l'acide 2-methy 2-[(1-oxo 2-propènyl) amino] 1-propanesulfonique sous forme acide libre ou partiellement ou totalement salifiée.

Selon un aspect particulier, le polyélectrolyte anionique réticulé (P) présent dans la composition (E2) comprend pour 100% molaire :
(a1) - une proportion supérieure ou égale à 25% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
(a2) - optionnellement, une proportion supérieure à 0% molaire et inférieure ou égale à 75% molaire, d'unités monomériques issues d'au moins un monomère choisi parmi les éléments du groupe constitué par l'acrylamide, le N,N-diméthyl acrylamide ; le méthacrylamide ou le N-isopropyl acrylamide ;
(a3) - optionnellement une proportion supérieure à 0% molaire et inférieure ou égale à 20% molaire, plus particulièrement supérieure à 0% molaire et inférieure ou égale à 15% molaire, encore plus particulièrement supérieure ou égale 0% molaire et inférieure ou égale à 10% molaire d'unités monomériques issues d'au moins un monomère choisi parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), et le vinyl pyrrolidone
(a4) - optionnellement une proportion supérieure à 0% molaire et inférieure ou égale à 75% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3- [(1-oxo 2-propènyl) amino] butanoïque, la fonction carboxylique desdits monomères étant sous forme acide libre, partiellement salifiée ou totalement salifiée ;
(a5) optionnellement une proportion supérieure à 0% molaire et inférieure ou égale à 5% molaire d'au moins un monomère de formule (1) : 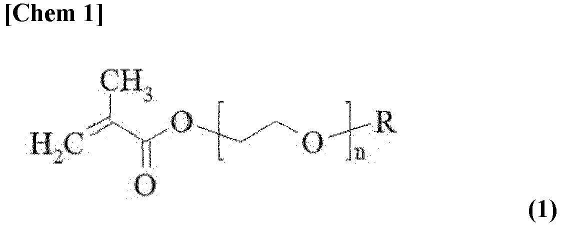 dans laquelle R représente un radical alkyle linéaire ou ramifié comportant de huit à vingt atomes de carbone et n représente un nombre entier supérieur ou égal à zéro et inférieur ou égal à vingt ;
(a6) - d'une proportion supérieure à 0% molaire et inférieure ou égale à 1% molaire d'unités monomériques issues d'au moins un monomère de réticulation (AR) diéthylénique ou polyéthylénique ; la somme des dites proportions molaires en unités monomériques selon a1), a2), a3), a4), a5) et a6) étant égale à 100% molaire.

Au sens de la présente invention, le terme «salifié» indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH₂-CH₂-NH₄⁺). Il s'agit de préférence des sels de sodium ou d'ammonium.

Par au moins un monomère de réticulation (AR) diéthylénique ou polyéthylénique, on désigne, dans la définition dudit polyélectrolyte anionique réticulé (P), on désigne notamment un monomère choisi parmi les éléments du groupe constitué par le méthylène-bis(acrylamide), le diméthacrylate d'éthylèneglycol, le diacrylate de diéthylèneglycol, le diacrylate d'éthylèneglycol, le diallyl urée, le triallylamine, le triméthylol propanetriacrylate, l'acide diallyoxyacétique ou un de ses sels comme le diallyloxyacétate de sodium, ou un mélange de ces composés ; et plus particulièrement un monomère choisi parmi le diméthacrylate d'éthylèneglycol, le triallylamine, le triméthylol propanetriacrylate ou le méthylène-bis(acrylamide) ou un mélange ces composés.

Selon un autre aspect particulier de la présente invention, la composition (E2) à usage topique se présentant sous la forme d'une émulsion de type eau-dans-huile, est caractérisée en ce que ledit monomère de réticulation (AR) tel que défini précédemment, est mis en oeuvre dans une proportion molaire inférieure ou égale à 0,5%, plus particulièrement inférieure ou égale à 0,25% et tout particulièrement inférieure ou égales à 0,1% ; elle est plus particulièrement supérieure ou égales à 0,005% molaire.

Le polyélectrolyte anionique réticulé (P) mis en oeuvre dans la composition (E2) peut également comprendre divers additifs, tels que des agents complexants, des agents de transfert ou des agents limiteurs de chaîne.

Le polyélectrolyte anionique réticulé (P) mis en oeuvre dans la composition (E2) peut être préparé par la mise en oeuvre d'un procédé de polymérisation radicalaire connu de l'homme du métier, comme par exemple les procédés de polymérisation en solution, de polymérisation en suspension, de polymérisation en suspension inverse, de polymérisation en émulsion, de polymérisation en émulsion inverse, de polymérisation en milieu solvant suivie d'une étape de précipitation du polymère formé.

Selon un aspect plus particulier, le polyélectrolyte anionique réticulé (P) mis en oeuvre dans la composition (E2) peut être préparé par la mise en oeuvre d'un procédé de polymérisation en milieu solvant suivie d'une étape de précipitation du polymère formé, ou de polymérisation en émulsion inverse optionnellement suivi d'une étape de concentration et/ou d'atomisation.

Selon un aspect plus particulier, le polyélectrolyte anionique réticulé (P) mis en oeuvre dans la composition la composition (E2) peut être préparé selon un des procédés décrits ci-dessus et impliquer l'utilisation d'agents de transfert ou limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en oeuvre, de 0,001% à 1% molaire, plus particulièrement de 0,001% à 0,5% molaire, et tout particulièrement de 0,001% à 0,1% molaire.

Selon un autre aspect particulier de la présente invention, ledit polyélectrolyte anionique réticulé (P) est un élément du groupe constitué par un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique partiellement ou totalement salifié sous forme de sel de sodium ou de sel d'ammonium et de l'acide acrylique partiellement ou totalement salifiés sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium ou de sel d'ammonium et d'acide acrylique (δ) partiellement ou totalement salifié sous forme de sel de sodium dans un rapport molaire (y)/(ô) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acide acrylique (δ)partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acrylamide (ε) dans un rapport molaire (γ)/(ε) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ) dans un rapport molaire (γ)/(ζ) supérieur ou égal à 30/70 et inférieur ou égal à 90/10, réticulé par le triallylamine et/ou le méthylène-bis(acrylamide) ; un terpolymère réticulé par le triallylamine et/ou le méthylènebis(acrylamide), de l'acide 2-méthyl 2-[(1-oxo 2-propènyl) amino] 1-propanesulfonique partiellement ou totalement salifié sous forme de sel de sodium ou de sel d'ammonium, de l'acrylamide et de l'acide acrylique partiellement ou totalement salifiés sous forme de sel de sodium ou de sel d'ammonium ; un terpolymère réticulé par le triallylamine et/ou le méthylène-bis(acrylamide), de l'acide 2-méthyl 2-[(1-oxo 2-propènyl) amino] 1- propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 45% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 10%, un terpolymère réticulé par le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 47% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 8% ; un terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5% ; un tétrapolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39 %, du méthacrylate de lauroyle dans une proportion molaire supérieure ou égale à 0,5% et inférieure ou égale à 2,5%, et du méthacrylate de stéaroyle dans une proportion molaire supérieure ou égale à 0,5% et inférieure ou égale à 2,5%.

Selon un aspect particulier, la composition (E2), est caractérisée en ce que l'agent tensioactif émulsionnant eau-dans-huile (S2) comprend au moins une composition (CA1) représentée par la formule (III) :

R₁-O-(G)ₓ-H (III)

dans laquelle x représente un nombre décimal compris entre 1,05 et 5, G représente le reste d'un sucre réducteur, et R₁ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (CA1) consistant en un mélange de composés représentés par les formules (III₁), (III₂), (III₃), (III₄) et (III₅) :

R₁-O-(G)₁-H (III₁)

R₁-O-(G)₂-H (III₂)

R₁-O-(G)₃-H (III₃)

R₁-O-(G)₄-H (III₄)

R₁-O-(G)₅-H (III₅)

dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical R₁ dans la formule (III) telle que définie ci-dessus :
- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (2) :

   (CH₃)(CH₃)CH-(CH₂)ᵣ-CH₂-OH (2)

   dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ;

Les radicaux alkyles ramifiés, issus des alcools de Guerbet, de formule (3) :

CH(CₛH₂ₛ₊₁)(CₜH₂ₜ₊₁)-CH₂-OH (3)

dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle.

Selon un aspect particulier, dans la définition de la formule (III) telle que définie ci-dessus, R₁ représente un radical aliphatique hydrocarboné, saturé ou insaturé, linéaire ou ramifié comportant de 12 à 24 atomes de carbone.

Par sucre réducteur, dans la définition de la formule (III) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique (G)ₓ, peut se présenter sous toutes formes d'isoméries, qu'il s'agisse d'isomérie optique, d'isomérie géométrique ou d'isomérie de position ; elle peut aussi représenter un mélange d'isomères.

Dans la formule (II) telle que définie ci-dessus, le groupe R₁-O- est lié à G par le carbone anomérique du reste saccharide, de manière à former une fonction acétal.

Selon un aspect particulier dans la définition de la formule (III) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi parmi les restes du glucose, du xylose et de l'arabinose.

Selon un aspect encore plus particulier, dans la définition de la formule (III) représentant la composition (CA1) comprise dans la composition (E₂) à usage topique se présentant sous la forme d'une émulsion de type eau-dans-huile et objet de la présente invention, x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, dans la définition de la formule (III) telle que définie ci-dessus, R₁ représente le radical choisi parmi au moins un des éléments du groupe constitué par les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Selon un aspect particulier, la composition (E2), est caractérisée en ce que l'agent tensioactif émulsionnant eau-dans-huile (S2) comprend au moins une composition (CA1) d'alkylpolyglycosides représentée par la formule (III) :

R1-O-(G)x-H (III)

dans laquelle x représente un nombre décimal compris entre 1,05 et 2,5, G représente le reste du xylose, et R₁ représente le radical 2-octyl dodécyle, ladite composition (C₁) consistant en un mélange de composés représentés par les formules (III₁), (III₂), (III₃), (III₄) et (III₅) :

R₁-O-(G)₁-H (III₁)

R₁-O-(G)₂-H (III₂)

R₁-O-(G)₃-H (III₃)

R₁-O-(G)₄-H (III₄)

R₁-O-(G)₅-H (III₅)

dans les proportions molaires respectives a₁, a₂, a₃, a₄ et a₅, telles que :
- La somme a₁₊ a₂ + a₃ + a₄ + a₅ est égale à 1 et que
- La somme a₁ + 2a₂ + 3a₃ + 4a₄ + 5a₅ est égale à x.

Par « G représente le reste du xylose » on entend « G représente le radical xylosyl ou α, β-D-xylopyranosyl, obtenu à partir de la suppression du groupe hydroxyl hémiacétal du β-D-xylopyranose ».

Selon un aspect particulier, la composition (E2) telle que définie précédemment, est caractérisée en ce que ledit agent émulsionnant (S2) consiste en une composition (CA2) comprenant pour 100% de sa masse :
- De 10 % à 50 % massique, de préférence de 15% à 40% massique, et encore plus préférentiellement de 20% à 30% massique de la composition d'alkylpolyglycosides (CA1)
   et
- De 90% à 50% massique, de préférence de 85% à 60% massique, et encore plus préférentiellement de 80% à 70% massique d'au moins un alcool gras de formule (IV) :

   R'₁-OH (IV),

   avec R'₁, identique ou différent de R₁, et représentant un radical aliphatique hydrocarboné, saturé ou insaturé, linéaire ou ramifié, éventuellement substitué avec un ou plusieurs groupe hydroxyle, comportant de 12 à 36 atomes de carbone.

Selon un aspect particulier, dans la définition de la formule (III) représentant la composition (CA1) comprise dans la composition (CA2), R₁ représente le radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle, G représente le reste d'un sucre réducteur choisi parmi les restes du glucose et du xylose et x représente un nombre décimal supérieur ou égal à 1,05 et inférieur ou égal à 2,5.

Selon un aspect plus particulier, dans la définition de la formule (III) représentant la composition (CA1) comprise dans la composition (CA2), R₁ représente le radical 2-octyl dodécyle, G représente le reste du xylose et x représente un nombre décimal supérieur ou égal à 1,05 et inférieur ou égal à 2,5.

Selon un aspect plus particulier, dans la définition de l'alcool gras de formule (IV) telle que définie ci-dessus, R'₁ représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle, R'₁ représente tout particulièrement le radical 2-octyl dodécyle.

Selon un aspect particulier, la composition (E2) telle que définie précédemment, est caractérisée en ce que ledit agent émulsionnant (S2) consiste en une composition (CA2) comprenant pour 100% de sa masse :
- De 10 % à 50 % massique de la composition d'alkylpolyglycosides (CA1) et

De 90% à 50% massique d'au moins un alcool gras de formule (IV) :

R'₁-OH (IV),

avec R'₁ représentant le radical 2-octyl dodécyle.

Selon un aspect particulier, la composition (E2) telle que définie précédemment, est caractérisée en ce que ledit agent émulsionnant (S2) consiste en une composition (CA3) comprenant pour 100% de sa masse :
- de 15 % à 25 % massique de la composition (CA1) et
- de 55 % à 65 % massique d'au moins un alcool gras de formule (IV) :

   R'1-OH (IV),

   dans laquelle R'1 représente le radical 2-octyl dodécyle ;
   de 10 % à 30 % massique d'au moins un polyhydroxystéarates de polyglycols représenté par la formule (V) : 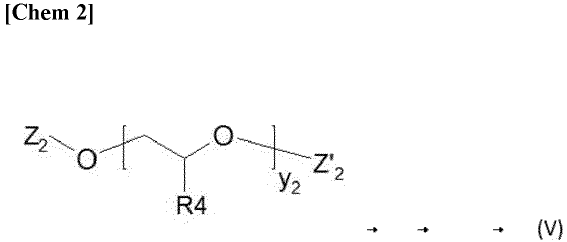 dans laquelle y2 représente un nombre entier supérieur ou égal à 2 et inférieur ou égal à 50, R4 représente l'atome d'hydrogène, le radical méthyle ou le radical éthyle, Z₂ représente un radical de formule (VI) : 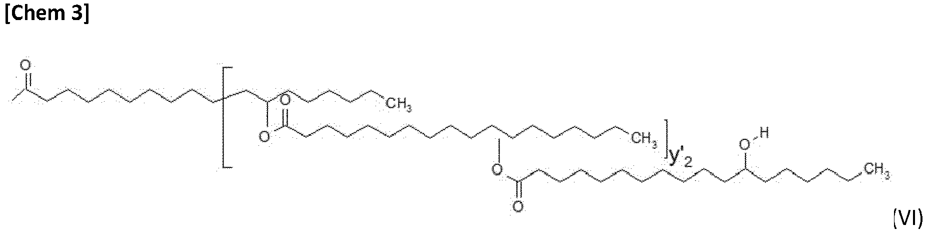 dans laquelle y'2 représente un nombre entier supérieur ou égal à 0 et inférieur ou égal à 10, plus particulièrement supérieur ou égal à 1 et inférieur ou égal à 10, Z'₂ représente un radical de formule (VI) telle que définie ci-dessus, avec Z₂' identique ou différent de Z₂, ou l'atome d'hydrogène.

Les compositions (C₁), (E1) et (E2) objets de la présente invention telles que définies précédemment sont destinées à un usage topique, et peuvent être incorporées dans tout type de formulation cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.).

Les compositions (C₁), (E1) et (E2) objets de la présente invention telles que définies précédemment peuvent être conditionnées sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on).

Les compositions (C₁), (E1) et (E2) objets de la présente invention telles que définies précédemment peuvent être utilisées comme laits nettoyants ou démaquillants, comme lotions nettoyantes ou démaquillantes, comme gels moussants pour le visage ou pour le corps, comme shampooing pour le nettoyage des cheveux et/ou du cuir chevelu, comme après-shampooing pour le traitement des cheveux et/ou du cuir chevelu, comme bain moussant, comme crème, comme lait ou comme lotion pour le soin ou pour la protection du visage, des mains et du corps, par exemple comme agent protecteur des rayonnements solaires, comme agent auto-bronzant, comme agent anti-âge, comme agent antirides, comme agent apaisant, comme agent hydratant.

Les compositions (C₁), (E1) et (E2) objets de la présente invention telles que définies précédemment peuvent en outre comporter des excipients et/ou des principes actifs habituellement mis en oeuvre dans le domaine des formulations à usage topique, en particulier cosmétiques, dermocosmétiques, pharmaceutiques ou dermo-pharmaceutique.

Les compositions (C₁), (E1) et (E2) objets de la présente invention telles que définies précédemment peuvent en outre comprendre un ou plusieurs composés auxiliaires choisi parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans les compositions (C₁), (E1) et (E2) objets de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer aux compositions (C₁), (E1) et (E2) objets de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents éventuellement présents dans les compositions (C₁), (E1) et (E2) objets de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans les compositions (C₁), (E1) et (E2) objets de la présente invention, on peut citer particulièrement les dérivés d'ammoniums quaternaires.

Parmi les tensioactifs non ioniques moussants et/ou détergents éventuellement présents dans les compositions (C₁), (E1) et (E2) objets de la présente invention, on peut citer plus particulièrement les alkylpolyglycosides comportant un radical aliphatique, linéaire ou ramifié, saturé ou insaturé, et comportant de 8 à 12 atomes de carbone ; les dérivés d'huile de ricin, les polysorbates, les amides de coprah, les N-alkylamines.

Comme exemples de tensioactifs épaississants et/ou gélifiants éventuellement présents dans les compositions (C₁), (E1) et (E2) objets de la présente invention, on peut citer :
- les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120 ;
- les esters gras alcoxylés tels que le PEG 150 pentaérythrytyl tétrastéarate commercialisé sous l'appellation CROTHIX^{™} DS53, le PEG 55 propylene glycol oléate commercialisé sous l'appellation ANTIL^{™} 141 ;
- les carbamates de polyalkylène glycols à chaînes grasses tels que le PPG 14 laureth isophoryl dicarbamate commercialisé sous l'appellation FLFACOS^{™} T211, le PPG 14 palmeth 60 hexyl dicarbamate commercialisé sous l'appellation ELFACOS^{™} GT2125.

Comme exemples d'agents déodorants éventuellement présents dans les compositions (C₁), (E1) et (E2) objets de la présente invention, on peut citer les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol, le caprylate de glycérol, le caprate de polyglycérol ; le 1,2-décanediol ; le 1,3-propanediol ; l'acide salicylique ; le bicarbonate de sodium ; les cyclodextrines ; les zéolithes métalliques ; le Triclosan^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents anti-oxydants éventuellement présents dans les compositions (C₁), (E1) et (E2) objets de la présente invention, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate).

Parmi les principes actifs que peuvent comprendre les compositions (C₁), (E1) et (E2) objets de la présente invention, on peut citer:
- les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SFPICALM^{™} S, l'allantoïne et le bisabolol ; les agents anti-inflammatoires ; les composés montrant une action hydratante comme l'urée, les hydroxyurées, le glycérolglucoside, le diglycérolglucoside, les polyglycérylglucosides, le glycérol, le diglycérol, le xylitylpolyglucoside commercialisé sous le nom de marque Aquaxyl^{™}; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM^{™}, l'ADIPOLESS^{™}, la fucoxanthine; les protéines N-acylées; les peptides N-acylés comme le MATRIXIL^{™}; les acides aminés N-acylés; les hydrolysâts partiels de protéines N-acylés; les acides aminés; les peptides; les hydrolysâts totaux de protéines; les extraits de soja, par exemple la Raffermine^{™}; les extraits de blé par exemple la TENSINE^{™} ou la GLIADINE^{™}; les extraits végétaux, tels que les extraits végétaux riches en tanins, les extraits végétaux riches en isoflavones ou les extraits végétaux riches en terpènes; les extraits d'algues d'eau douce ou marines; les extraits de plantes marines; les extraits marins en général comme les coraux; les cires essentielles; les extraits bactériens; les céramides; les phospholipides; les composés montrant une action antimicrobienne ou une action purifiante, comme le LIPACIDE^{™} C8G, le LIPACIDE^{™} UG, le SEPICONTROL^{™} A5; l'OCTOPIROX^{™} ou le SENSIVA^{™} SC50; les composés montrant une propriété énergisante ou stimulante comme le PHYSIOGENYL^{™}, le panthénol et ses dérivés comme le SEPICAP^{™} MP; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™}; le SURVICODE^{™}; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683;; les agents connus pour leur action de facilitation et/ou d'accélération du bronzage et/ou du brunissement de la peau humaine, et/ou pour leur action de coloration de la peau humaine, comme par exemple les caraténoïdes ( et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylène glycol and Acetyl Tyrosine and hydrolyzed vegetable protein and Adenosine triphosphate) par la société UNIPEX, le produit commercialisé sous le nom de marque « Try-Excell^{™} » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI: hydrolyzed wheat protein and acetyl tyrosine and copper gluconate) par la société Alban Muller, le produit commercialisé sous le nom de marque Tyrostan^{™} (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze^{™} (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Actives, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI: Butylène glycol, Palmitoyl Tripeptide-40) par la société LIPOTEC, les sucres et les dérivés de sucres comme par exemple le produit commercialisé sous le nom de marque Tanositol^{™} (nom INCI: inositol) par la société Provital, le produit commercialisé sous le nom de marque Thalitan^{™} (ou Phycosaccharide^{™} AG) par la société CODIF international (nom INCI: Aqua and hydrolyzed algin (Laminaria Digitata) and magnésium sulfate and manganèse sulfate) contenant un oligosaccharide d'origine marine (acide guluronique et acide mannuronique chélatés avec les ions magnésium et manganèse), le produit commercialisé sous le nom de marque Melactiva^{™} (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines).

Parmi les agents de texture que peuvent comprendre les compositions (C₁), (E1) et (E2) objets de la présente invention, on peut citer la lauroyl lysine commercialisée sous l'appellation AMINOHOPE^{™}LL par la société AJINOMOTO, l'octenyl starch succinate commercialise sous l'appellation DRYFLO^{™} par la société NATIONAL STARCH, le myristyl polyglucoside commercialisé par SEPPIC sous l'appellation MONTANOV^{™} 14, les fibres de cellulose, les fibres de coton, les fibres de chitosane.

Le procédé de préparation de la composition (E₂) telle que définie précédemment, comprend les étapes suivantes:
Une étape a) de préparation de la phase grasse (G₂) en mélangeant l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 50 tours/minute et inférieure ou égale à 100 tours/minute ;
Une étape b) de préparation de la phase aqueuse (A₂) de l'ensemble des éléments la constituant dans les proportions souhaitées. Cette étape de mélange est généralement conduite à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C ; elle est réalisée sous agitation mécanique à une vitesse modérée supérieure ou égale à 500 tours/minute et inférieure ou égale à 3 000 tours/minute. De façon particulière, la phase aqueuse (A₁) obtenue à l'issue de l'étape b), présente une viscosité dynamique, mesurée à 20°C par l'intermédiaire d'un viscosimètre de type Brookfield LV à une vitesse de 6 tours/minute, supérieure ou égale à 200 mPa.s et inférieure ou égale à 40 000 mPa.s, plus particulièrement supérieure ou égale à 1 000 mPa.s et inférieure ou égale à 40 000 mPa.s, et encore plus particulièrement supérieure ou égale à 2 000 mPa.s et inférieure ou égale à 40 000 mPa.s ;
Une étape c) au cours de laquelle la phase grasse (G₂) est ajoutée sur la phase aqueuse (A₁) à une température supérieure ou égale à 20°C et inférieure ou égale à 80°C, plus particulièrement supérieure ou égale à 20°C et inférieure ou égale à 60°C, et encore plus particulièrement supérieure ou égale à 20°C et inférieure ou égale à 40°C, sous agitation mécanique à une vitesse modérée supérieure ou égale à 50 tours/minute et inférieure ou égale à 400 tours/minute, de façon à obtenir la composition (E₂).
Les exemples suivants illustrent l'invention sans toutefois la limiter.

### A- Préparation de compositions selon l'invention et de compositions comparatives, comprenant un mélange (M₁) et des esters.

On introduit la quantité désirée de la composition commercialisée sous le nom de marque Emogreen^{™}L19 dans un réacteur en verre muni d'une double enveloppe dans laquelle circule un fluide thermostaté et d'une agitation mécanique munie d'un agitateur équipé d'une pâle de type ancre. La vitesse d'agitation est réglée à 50 tours/minute et la température à 25°C. La quantité désirée d'ester de formule (I) ou de formule (II) est ensuite introduite progressivement et le mélange ainsi obtenu est homogénéisé pendant 30 minutes à une température de 25°C et à une vitesse d'agitation de 80 tours/minute. Le mélange est ensuite vidangé.

De la sorte, on obtient les compositions C11 et C12 selon l'invention et les compositions C21, C22 et C23 comparatives, dont les constitutions massiques sont décrites dans le tableau 1 ci-dessous.
Compositions C11 et C12 selon l'invention et les compositions C21, C22 et C23 comparatives

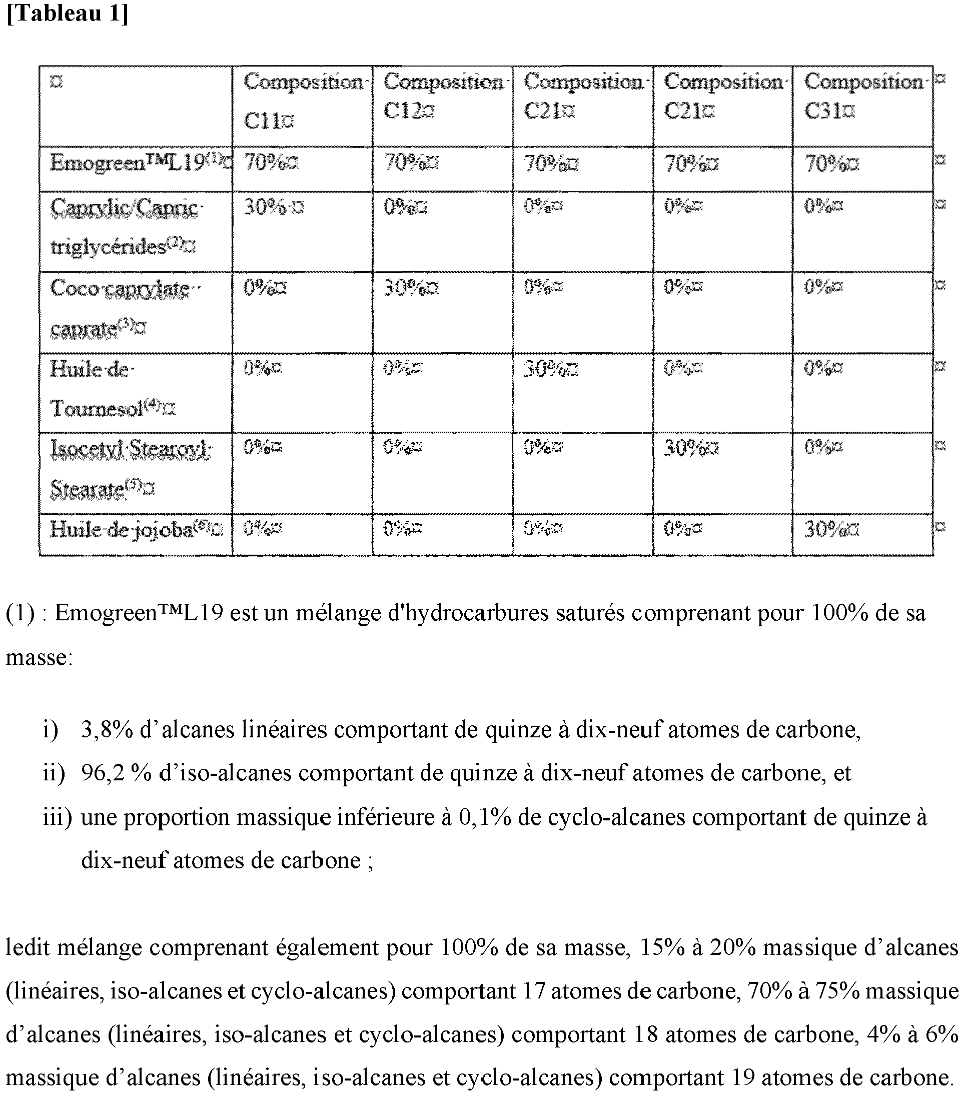

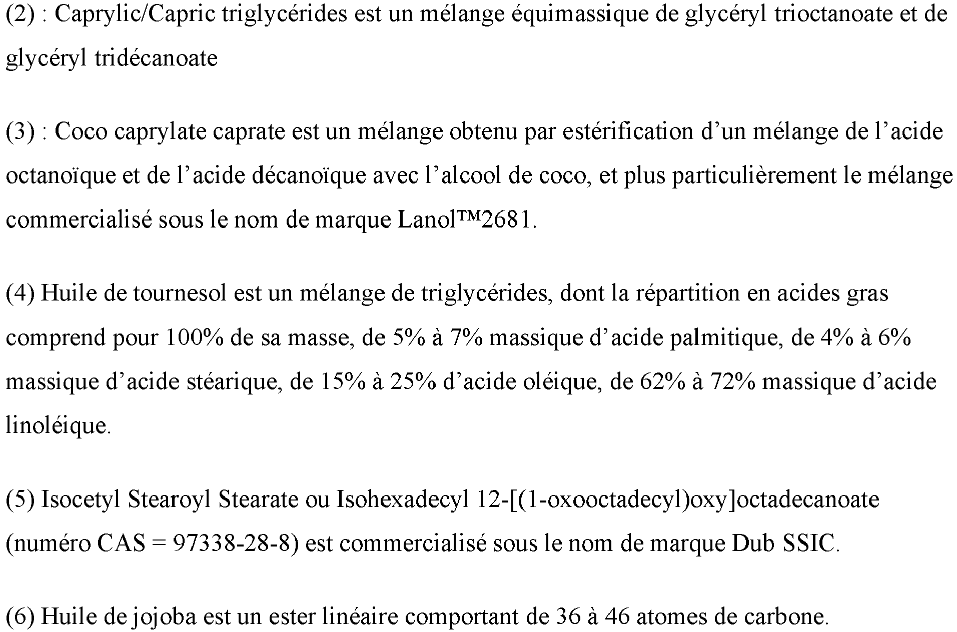

### B- Evaluation de la stabilité des compositions C11, C12 selon l'invention et des compositions comparatives C21, C22 et C23

a) On introduit dans une enceinte climatique régulée à 25°C une quantité de 100 mL de la composition à tester et contenue dans un flacon de 250 mL, pendant une durée de 3 mois.
   On évalue avant la mise en stabilité dans l'enceinte et après une durée de trois mois dans ladite enceinte climatique, les critères suivants :
   - Aspect visuel de la composition testée
   - Odeur de la composition testée
b) On introduit dans une enceinte climatique régulée à 45°C une quantité de 100 mL de la composition à tester et contenue dans un flacon de 250 mL, pendant une durée de 3 mois.
   On évalue avant la mise en stabilité dans l'enceinte et après une durée de trois mois dans ladite enceinte climatique, les critères suivants :
   - Aspect visuel de la composition testée
   - Odeur de la composition testée
c) Les résultats obtenus sont consignés dans le tableau 2 suivant :
   Evaluation organoleptique des compositions C11 et C12 selon l'invention et les compositions C21, C22 et C23 comparatives.

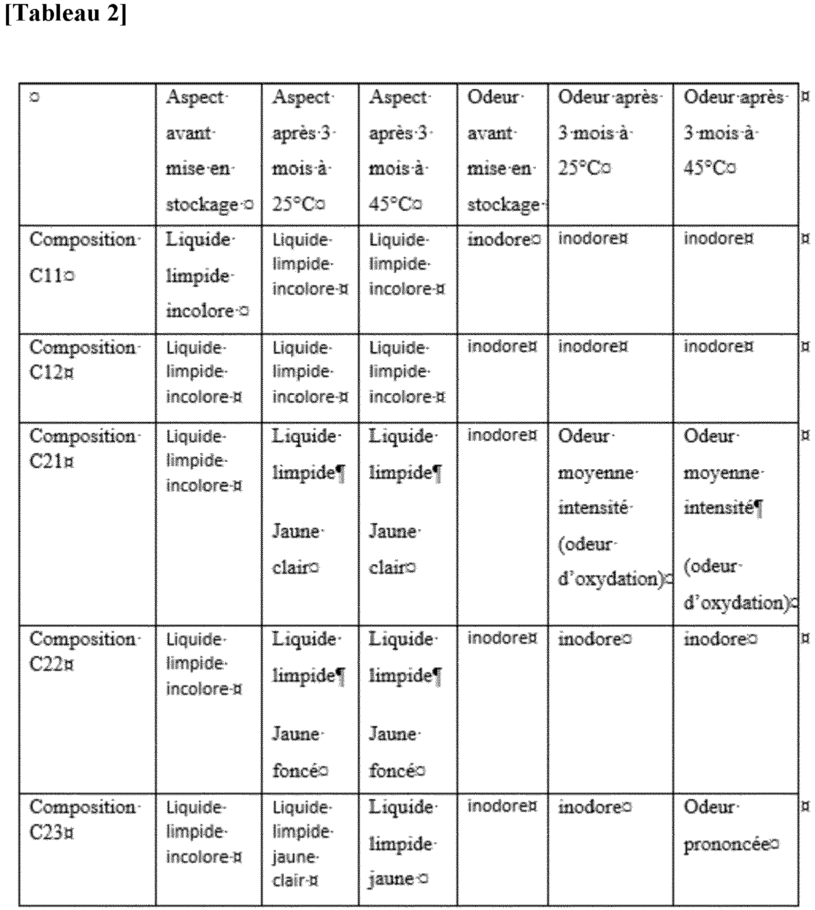

Les résultats obtenus lors de l'évaluation des propriétés organo-leptiques, notamment lors d'un stockage prolongé de trois mois à 45°C, les compositions selon l'invention restent inodores et incolores, alors que les compositions comparatives présentent une odeur prononcée et/ou l'apparition d'une couleur jaune indésirable dans le cadre d'une utilisation ultérieure pour la préparation d'une composition cosmétique complexe.

### C- Préparation d'émulsions huile-dans-eau selon l'invention, comprenant les compositions selon l'invention et des compositions comparatives

On prépare une émulsion huile-dans-eau selon l'invention, notée (F₁₁), dont les proportions massiques de ses constituants sont indiquées dans le tableau 3, et trois émulsions huile-dans-eau comparatives notées (F₂₁) à (F₂₃) dont les proportions massiques de leurs constituants sont indiquées dans le tableau 3 ci-dessous. Le procédé de préparation commun pour les émulsions huile-dans-eau selon l'invention et pour les émulsions huile-dans-eau comparatives, est le suivant:
- On verse dans un bécher, à une température de 20°C, la composition à tester, sous agitation mécanique à 80 tours/minute;
- On y ajoute ensuite, la quantité requise de l'agent épaississant SEPINOV^{™}EMT 10 sous agitation mécanique à 80 tours/minute et à 20°C;
- On prépare par mélange dans un bécher, à une température de 20°C, la phase aqueuse comprenant l'eau et l'Euxyl^{™}PE9010,
- Le mélange alors obtenu est refroidi sous agitation de type défloculeuse à 1500 tours par minute pendant vingt minutes, puis vidangé pour obtenir l'émulsion huile-dans-eau souhaitée.

**[Tableau 3]**

| | **Emulsion** | | | |
|---|---|---|---|---|
| | **(F₁₁)** | **(F₂₁)** | **(F₂₂)** | **(F₂₃)** |
| **Phase grasse** | | | | |
| Sepinov^{™}EMT10⁽⁷⁾ | 1,5% | 1,5% | 1,5% | 1,5% |
| Emogreen^{™}L19⁽¹⁾ | 0% | 10% | 0% | 0% |
| Lanol2681^{™(3)} | 0% | 0%% | 10% | 0% |
| Composition C11 | 10% | 0% | 0% | 0% |
| Heptyl Undecylenate (and) C13-C16 Isoparaffin | 0% | 0% | 0% | 10% |

| **Phase aqueuse** | | | | |
|---|---|---|---|---|
| Eau | Qs. 100% | Qs. 100% | Qs. 100% | Qs. 100% |
| Euxyl^{™}PE 9010⁽⁸⁾ | 1% | 1% | 1% | 1% |

(7) (Sepinov^{™}EMT10): Agent épaississant (nom INCI: hydroxyethyl acrylate / acryloyldimethyltaurate acrylate copolymer),
(8) (Euxyl^{™}PE9010 est un agent conservateur.

### D- Evaluation des propriétés sensorielles d'émulsions huile-dans-eau selon l'invention et d'émulsions huile-dans-eau comparatives

### Principe de la méthode

Treize panélistes dûment entraînés et habilités ont évalué les critères de "collant" lors de l'étalement sur la peau de l'émulsion testée, de "brillance de la peau" après étalement de l'émulsions testée, pour des émulsions huile-dans-eau selon l'invention et d'émulsions huile-dans-eau comparatives, en prenant comme base de référence l'émulsion (F11).

### Mode opératoire

Le mode opératoire mis en oeuvre comprend 5 étapes qui sont les suivantes :
- Etape 1 contrôle et évaluation de l'aspect et de l'odeur de l'émulsion huile-dans eau testée,
- Etape 2 : prise en main : évaluation de la facilité de préhension, et observation d'un éventuel aspect filant,
- Etape 3 : étalement sur la peau de l'émulsion testée par application circulaire à la surface de la peau, à une même vitesse pendant 10 tours, et recueil des sensations perçues à la fin du 10^{ème} tour,
- Etape 4 : poursuite de l'étalement de l'émulsion testée sur la peau toujours par application du même mouvement circulaire jusqu'à observation de l'absence de film d'émulsion sur la peau et recueil des sensations perçues,
- Etape 5 : recueil des sensations perçues après 1 minute après la fin de l'étalement.
Ce mode opératoire est mis en oeuvre à une température de 20°C.

### Expression des résultats

Pour chaque émulsion testée, et pour chaque critère sensoriel évalué, chaque panéliste indique si ladite émulsion testée procure une sensation améliorée par rapport à l'émulsion (F₁₁) de référence. L'ensemble des évaluations est collecté et les données sont traitée de façon statistique de façon à déterminer le caractère significatif d'une éventuelle différence, amélioration ou dégradation, entre la sensation perçue pour l'émulsion testée et l'émulsion de référence.

### Résultats

### Critère de "collant"

- Emulsion de référence : émulsion (F₁₁).

Les émulsions comparatives (F₂₁) à (F₂₃) sont évaluées selon le protocole défini ci-dessus et les résultats obtenus sont consignés dans le tableau 4 ci-dessous. L'augmentation de la sensation de collant par rapport à (F₁₁) est notée ">(F₁₁)" et la diminution de la sensation de collant par rapport à (F₁₁) est notée "<(F₁₁)" à chaque moment du processus d'étalement.

**[Tableau 4]**

| | | | |
|---|---|---|---|
| Etape 3: Evaluation après les 10 premiers tours | <(F₁₁) | >(F₁₁) | >(F₁₁) |
| Etape 4: Evaluation après pénétration | <(F₁₁) | >(F₁₁) | >(F₁₁) |
| Etape 5: Evaluation 1 minute après fin de l'étalement | <(F₁₁) | >(F₁₁) | >(F₁₁) |

### Critère de "brillance de la peau"

- Emulsion de référence : émulsion (F₁₁)
Les émulsions comparatives (F₂₁) à (F₂₃) sont évaluées selon le protocole défini ci-dessus et les résultats obtenus sont consignés dans le tableau 4 ci-dessous. L'augmentation de la brillance de la peau par rapport à (F₁₁) est notée ">(F₁₁)" et sa diminution notée "<(F₁₁)".

**[Tableau 5]**

| | | | |
|---|---|---|---|
| Etape 3: Evaluation après les 10 premiers tours | >(F₁₁)* | <(F₁₁) | <(F₁₁) |
| Etape 4: Evaluation après pénétration | >(F₁₁)* | <(F₁₁) | <(F₁₁) |
| Etape 5: Evaluation 1 minute après fin de l'étalement | >(F₁₁)* | <(F₁₁) | <(F₁₁) |

| | | | |
|---|---|---|---|
| (*) : observation d'un résidu huileux sur la peau. | | | |

### Analyse des résultats

Les résultats obtenus montrent une amélioration des propriétés sensorielles pour l'émulsion F(₁₁), en comparaison avec les émulsions (F₂₁) à (F₂₃), qui permet disposer d'une émulsion avec une sensation de collant après étalement diminuée et de diminuer la brillance de la peau après étalement.

## Revendications

1. Composition (C1) comprenant pour 100% de sa masse :
a) de 30% à 80% massique d'au moins un ester choisi parmi les éléments du groupe constitué par :
- les composés de formule (I) :
CH₃- (CH₂)ₓ₁- C(=O)-O-CH₂-CH[O-C(=O)-(CH₂)ₓ₂-CH₃]-CH₂-O-C(=O)-(CH₂)ₓ₃-CH₃ (I)
avec x₁, x₂ et x₃ représentant un nombre entier compris entre 6 et 8, et par
- les composés de formule (II) :
R-C(=O)-O-R' (II)
avec :
- R-C(=O) représente un radical acyle linéaire et saturé, comportant de huit à dix atomes de carbone et
- R' représente un radical alkyle linéaire et saturé, comportant de huit à dix-huit atomes de carbone,
et
b) de 20% massique à 70% massique d'un mélange (M1) d'hydrocarbures parmi lesquels au moins 94% massique comportent de quinze à dix-neuf atomes de carbone, ledit mélange (M1) comprenant pour 100% de sa masse:
- Une proportion massique en alcanes ramifiés supérieure ou égale à 90% et inférieure ou égale à 100%;
- Une proportion massique en alcanes linéaires supérieure ou égale à 0% et inférieure ou égale à 10%;
- Une proportion massique en cyclo-alcanes supérieure ou égale à 0% et inférieure ou égale à 1%.

2. Composition (C1) selon la revendication 1, **caractérisée en ce que** ledit au moins un ester choisi parmi les éléments du groupe constitué par les composés de formule (I) et les composés de formule (II), est choisi parmi les éléments du groupe constitué par le glycéryl trioctanoate (numéro CAS = 538-23-8), le glycéryl tridécanoate (numéro CAS = 621-71-6), un mélange de glycéryl trioctanoate et de glycéryl tridécanoate et le produit de la réaction d'estérification d'un mélange équimolaire d'acide décanoïque et d'acide octanoïque avec l'alcool de coco.

3. Composition C1 selon la revendication 2, **caractérisée en ce que** ledit au moins un ester choisi parmi les éléments du groupe constitué par les composés de formule (I) et les composés de formule (II), est choisi parmi le mélange équimassique de glycéryl trioctanoate (numéro CAS = 538-23-8) et de glycéryl tridécanoate (numéro CAS = 621-71-6), et le produit commercialisé sous le nom INCI « Coco-caprylate/caprate » (numéro CAS = 95912-86-0).

4. Composition (C1) selon l'une quelconque des revendications 1 à 3, **caractérisée en ce que** ledit mélange (M1) comprend pour 100% de sa masse :
i) 3,8% d'alcanes linéaires comprenant de quinze à dix-neuf atomes de carbone,
ii) 96,2% d'iso-alcanes comprenant de quinze à dix-neuf atomes de carbone, et
iii) une proportion massique inférieure à 0,1% de cycloalcanes comprenant de quinze à dix-neuf atomes de carbone,
et **en ce que** ledit mélange (M1) comprend aussi pour 100% de sa masse, 15% à 20% massique d'alcanes (linéaires, iso-alcanes et cycloalcanes ) comportant 17 atomes de carbone, 70% à 75% massique d'alcanes (linéaires, iso-alcanes et cycloalcanes ) comportant 18 atomes de carbone et 4% à 6% massique d'alcanes (linéaires, iso-alcanes et cycloalcanes ) comportant 19 atomes de carbone.

5. Composition (C1) **caractérisée en ce qu'**elle comprend pour 100% de sa masse :
a) de 30% à 80% massique d'au moins un ester choisi parmi le mélange équimassique de glycéryl trioctanoate (numéro CAS = 538-23-8) et de glycéryl tridécanoate (numéro CAS = 621-71-6), et le produit commercialisé sous le nom INCI « Coco-caprylate/caprate » (numéro CAS = 95912-86-0), et
b) de 20% massique à 70% massique dudit mélange (M1) comprenant pour 100% de sa masse :
i) 3,8% d'alcanes linéaires comprenant de quinze à dix-neuf atomes de carbone,
ii) 96,2% d'iso-alcanes comprenant de quinze à dix-neuf atomes de carbone, et
iii) une proportion massique inférieure à 0,1% de cycloalcanes comprenant de quinze à dix-neuf atomes de carbone,
et comprenant aussi pour 100% de sa masse, 15% à 20% massique d'alcanes (linéaires, iso-alcanes et cycloalcanes ) comportant 17 atomes de carbone, 70% à 75% massique d'alcanes (linéaires, iso-alcanes et cycloalcanes ) comportant 18 atomes de carbone et 4% à 6% massique d'alcanes (linéaires, iso-alcanes et cycloalcanes ) comportant 19 atomes de carbone.

6. Composition (C1) selon la revendication 5 **caractérisée en ce qu'**elle comprend pour 100% de sa masse :
a) 30% massique d'au moins un ester de formule (I) choisi parmi le mélange équimassique de glycéryl trioctanoate (numéro CAS = 538-23-8) et de glycéryl tridécanoate (numéro CAS = 621-71-6), et le produit commercialisé sous le nom INCI « Coco-caprylate/caprate » (numéro CAS = 95912-86-0), et
b) 70% massique dudit mélange (M1) comprenant pour 100% de sa masse :
i) 3,8% d'alcanes linéaires comprenant de quinze à dix-neuf atomes de carbone,
ii) 96,2% d'iso-alcanes comprenant de quinze à dix-neuf atomes de carbone, et
iii) une proportion massique inférieure à 0,1% de cycloalcanes comprenant de quinze à dix-neuf atomes de carbone,
et comprenant aussi pour 100% de sa masse, 15% à 20% massique d'alcanes (linéaires, iso-alcanes et cycloalcanes ) comportant 17 atomes de carbone, 70% à 75% massique d'alcanes (linéaires, iso-alcanes et cycloalcanes ) comportant 18 atomes de carbone et 4% à 6% massique d'alcanes (linéaires, iso-alcanes et cycloalcanes ) comportant 19 atomes de carbone.

7. Composition (C1) selon la revendication 6, **caractérisée en ce que** ledit au moins un ester de formule (I) est le mélange équimassique de glycéryl trioctanoate (numéro CAS = 538-23-8) et de glycéryl tridécanoate (numéro CAS = 621-71-6).

8. Composition (C1) selon la revendication 6, **caractérisée en ce que** ledit au moins un ester de formule (I) le produit commercialisé sous le nom INCI « Coco-caprylate/caprate » (numéro CAS = 95912-86-0).

9. Composition (E1) à usage topique se présentant sous la forme d'une émulsion de type huile-dans-eau, **caractérisée en ce qu'**elle comprend pour 100% de sa masse :
a) De 50% à 90% massique, d'une phase aqueuse (A1) cosmétiquement acceptable,
b) De 10% à 50% massique, d'une phase grasse (G1) comprenant pour 100% de sa masse :
b₁₁) De 10% à 80% massique d'une composition (C₁) telle que définie à l'une ou quelconque des revendications 1 à 8 ;
b₁₂) De 0,5% à 20% massique, d'au moins un agent tensioactif de type huile-dans-eau (S1),
b₁₃) De 0% à 89,5% massique, d'au moins une huile et/ou une cire de composition différente de la composition (C₁),
avec la somme des proportions massiques de b₁₁), b₁₂) et b₁₃) égale à 100% de la phase grasse (G1).

10. Composition (E1) selon la revendication 9, **caractérisée en ce que** la phase grasse (G1) comprend:
b₁₄) De 5% à 30% massique, d'au moins un agent de protection contre les rayonnements ultra-violets du soleil, avec la somme des proportions massiques de b₁₁), b₁₂), b₁₃) et b₁₄) égale à 100% de la phase grasse (G1).

11. Composition (E2) à usage topique se présentant sous la forme d'une émulsion de type eau-dans-huile, **caractérisée en ce qu'**elle comprend pour 100% de sa masse :
a) De 60% à 98% massique, d'une phase aqueuse (A2) cosmétiquement acceptable,
b) De 2% à 40% massique, d'une phase grasse (G2) comprenant pour 100% de sa masse :
b₂₁) De 10% à 80% massique d'une composition (C₁) telle que définie à l'une ou quelconque des revendications 1 à 8 ;
b₂₂) De 0,5% à 20% massique, d'au moins un agent tensioactif de type eau-dans-huile (S2),
b₂₃) De 0% à 89,5% massique, d'au moins une huile et/ou une cire de composition différente de la composition (C₁), et
avec la somme des proportions massiques de b₂₁), b₂₂) et b₂₃) égale à 100% de la phase grasse (G2).

12. Composition (E2) à usage topique se présentant sous la forme d'une émulsion de type eau-dans-huile et telle que définie à la revendication 11, **caractérisée en ce que** la phase aqueuse (A2) comprend pour 100% de sa propre masse de 0,5% massique à 10% massique d'un moins un polymère polyélectrolyte anionique réticulé (P).

13. Composition (E2) selon l'une quelconque des revendications 11 ou 12, **caractérisée en ce que** la phase grasse (G2) comprend:
b₁₄) De 5% à 30% massique, d'au moins un agent de protection contre les rayonnements ultra-violets du soleil, avec la somme des proportions massiques de b₂₁), b₂₂), b₂₃) et b₂₄) égale à 100% de la phase grasse (G2).

14. Utilisation d'une composition (E1) selon l'une des revendications 9 ou 10, ou d'une composition (E2) selon l'une des revendications 11 à 13, pour protéger la peau humaine contre les effets inesthétiques de l'exposition aux rayonnements ultra-violets du soleil, et plus particulièrement contre les rougeurs.

15. Composition (E1) selon l'une des revendications 9 ou 10, ou composition (E2) selon l'une des revendications 11 à 13, pour traiter ou ralentir une altération de la peau due à des brûlures, des coups de soleil, des érythèmes ou des cancers.

16. Composition (E2) selon l'une quelconque des revendications 11 ou 12, **caractérisée en ce que** la phase grasse (G2) comprend:
b₁₄) De 5% à 15% massique, d'au moins un pigment coloré, avec la somme des proportions massiques de b₂₁), b₂₂), b₂₃) et b₂₄) égale à 100% de la phase grasse (G2).
